# EUROPEAN PATENT SPECIFICATION

(11) **EP 2 296 748 B1**
(45) Date of publication and mention of the grant of the patent: **02.04.2014**
(21) Application number: 08824894.3
(22) Date of filing: 08.10.2008
(51) Int. Cl.: A61F 13/00, A61K 9/70, A61K 31/4535

(54) **KETOTIFEN TRANSDERMAL DRUG DELIVERY SYSTEMS AND METHODS FOR TREATING OPHTHALMIC DISEASE**
TRANSDERMALE ARZNEIMITTELABGABESYSTEME FÜR KETOTIFEN UND VERFAHREN ZUR BEHANDLUNG VON AUGENERKRANKUNGEN
SYSTÈMES DE DÉLIVRANCE DE MÉDICAMENT TRANSDERMIQUE DE KÉTOTIFÈNE ET PROCÉDÉS POUR TRAITER UNE MALADIE OPHTALMIQUE

(30) Priority: 30.05.2008 US 57748 P; 23.09.2008 US 236392
(43) Date of publication of application: 23.03.2011
(73) Proprietor: SENJU USA, INC., Woodland Hills, CA 91367 (US)
(72) Inventor: PONGPEERAPAT, Adchara, San Jose, California 95131 (US); YAMAJI, Masahiro, San Jose California 95131 (US)
(74) Representative: von Kreisler Selting Werner
(86) International application number: PCT/US2008/079211
(87) International publication number: WO 2009/145801

(56) References cited:
- EP-A1- 1 074 251
- EP-A1- 1 541 176
- US-A1- 2002 012 695
- US-A1- 2006 036 220
- US-A1- 2006 257 460
- English machine translation of JP2011-74034

## Description

### INTRODUCTION

Transdermal drug delivery systems, also known as transdermal patches or skin patches, are medicated adhesive patches that are placed on the skin to deliver medication through the skin. Transdermal patches deliver medication by percutaneous absorption, which is the absorption of substances through unbroken skin. After a transdermal patch is applied to the skin, the medication contained in the patch passes through, or permeates, the skin and can reach its site of action through a systemic blood flow. Alternatively, the transdermal patch may be placed on the desired treatment site such that the medication contained in the patch is delivered topically.

Ketotifen (4,9-dihydro-4-(1-methyl-4-piperidinylidene)- 10H-benzocyclohepta[1,2-b]thiopen-10-one) is an antihistamine and mast cell stabilizer that is used to treat allergic conjunctivitis, or itchy red eyes caused by allergies. Typically, ketotifen is provided as a solution of Ketotifen fumarate (brand name solutions include Zaditor from Novartis and Alaway from Bausch and Lomb). Inviduals use solution formulations of Ketotefin fumarate as eye drops to treat allergic symptoms.

US-A1-2002/0012695 discloses in a patch comprising ketotifen fumarate and an adhesive consisting of a copolymer of 2- ethylhexyl acrylate, methylmethacrylate and vinyl acetate.

### SUMMARY

A ketotefin transdermal drug delivery system is provided. In certain embodiments, the system includes a support layer and a plaster layer provided on the support, wherein the plaster layer contains ketotifen freebase. Also provided are methods of using the transdermal drug delivery systems, e.g., for treatment of ophthalmic diseases. Also provided are kits containing the transdermal drug delivery system for use in the subject methods.

### BRIEF DESCRIPTION OF THE FIGURES

Figure 1 shows a graph of permeation amount (µg/cm²) vs. time (hours) for rat in vitro permeation tests using Example formulations 1 and 2.
Figure 2 shows a graph of permeation amount (µg/cm²) vs. time (hours) for rat in vitro permeation tests of Example formulation 1 of an embodiment of the invention and a comparative example formulation 3.

### DEFINITIONS

The terms "plaster" and "plaster layer" mean a solid or semi-solid preparation spread on the surface of a support, such as cloth, plastic, or other material, and applied to a skin surface of a subject.

The terms "pressure-sensitive adhesive", "self adhesive", and "self-stick adhesive" mean an adhesive that forms a bond when pressure is applied to adhere the adhesive with a surface. Typically, no solvent, water, or heat is needed to activate the adhesive. For pressure-sensitive adhesives, the degree of bond strength is proportional to the amount of pressure that is used to apply the adhesive to the surface.

The terms "skin surface" and "surface of skin" mean the outer surface of the skin, including the skin surfaces of the front surface of the eyelid, such as but not limited to, the skin surfaces of the front surfaces of the upper and lower eyelids, and the skin surfaces of the front surfaces of the upper and lower eyelids and in the vicinity thereof. The front surface of the eyelid is covered with the skin, while the rear surface thereof is covered with the conjunctiva.

### DETAILED DESCRIPTION

A ketotifen transdermal drug delivery system is provided. In certain embodiments, the system includes a support layer and a plaster layer provided on the support, wherein the plaster layer contains ketotifen freebase. Also provided are methods of using the transdermal drug delivery systems, e.g., for treatment of ophthalmic diseases. Also provided are kits containing the transdermal drug delivery system for use in the subject methods.

Before the present invention is described in greater detail, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

As will be apparent to those of skill in the art upon reading this disclosure, each of the individual embodiments described and illustrated herein has discrete components and features which may be readily separated from or combined with the features of any of the other several embodiments without departing from the scope or spirit of the present invention. Any recited method can be carried out in the order of events recited or in any other order which is logically possible.

In further describing various embodiments of the invention, aspects of the transdermal drug delivery systems are reviewed first in greater detail, followed by a detailed description of embodiments of using the transdermal delivery systems and a review of kits that include the transdermal delivery systems.

### KETOTIFEN TRANSDERMAL DRUG DELIVERY SYSTEMS

As summarized above, ketotefin transdermal drug delivery systems are provided. Transdermal delivery systems of the invention are formulations or compositions that are configured to deliver an active agent, specifically ketotefin, to a subject when topically applied to a skin surface of a subject. Transdermal drug delivery systems of the invention may have one or more layers. In certain embodiments, the transdermal drug delivery systems may have a support layer and a plaster layer, where the plaster layer is present on the support layer. The plaster layer may contain an active agent for ophthalmic diseases. In some embodiments, the active agent for ophthalmic diseases includes, but is not limited to ketotifen freebase. Ketotifen free base has the formula: C₁₉H₁₉NOS and the IUPAC name 4,9-dihydro-4-(1-methyl-4-piperidinylidene)-10*H*-benzo[4,5]cyclohepta[1,2-b]thiophen-10-one. Ketotifen is described by the formula:

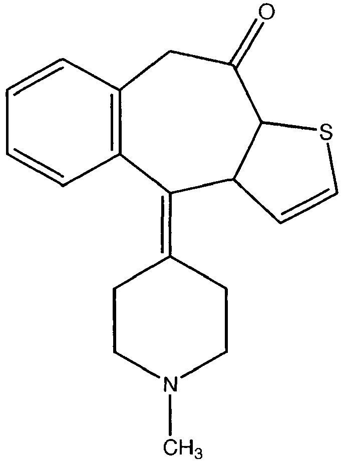

An aspect of the subject delivery systems is that they are storage stable. By storage-stable is meant that the compositions may be stored for extended periods of time without significant degradation and/or significant reduction in activity of the ketotifen active agent. In certain embodiments, the subject compositions are stable for 3 years or longer, etc., when maintained at 25°C.

In some cases, the ratio of the amount of ketotifen freebase in the system to the initial amount of ketotifen freebase in the system after storage at about 60 °C for at least one month is 92% or more, 93% or more, such as 94% or more, including 95% or more, or greater. In some embodiments, the ratio of the amount of ketotifen freebase in the system to the initial amount of ketotifen freebase in the system is 92% or greater after storage at about 60 °C for at least one month as determined using the assay protocol reported in the experimental section, below.

An aspect of the subject delivery systems is that they provide for effective transdermal delivery of ketotifen. By the phrase "effective transdermal delivery of ketotifen" is meant that application of the transdermal drug delivery system to the surface of skin results in the transfer of a sufficient amount of ketotifen through the skin to produce the desired efficacy. In certain embodiments, the permeation amount of ketotifen freebase after about 12 hours is 1 µg/cm² or greater, 2 µg/cm² or greater, such as 3 µg/cm² or greater, including 4 µg/cm² or greater, e.g., 5 µg/cm² or greater, e.g., 10 µg/cm² or greater, etc. In some cases, the permeation amount of ketotifen freebase is 1 µg/cm² or greater after about 12 hours as determined using the assay protocol reported in the experimental section, below.

In certain embodiments, the transdermal drug delivery system may be provided in the form of an adhesive tape or an adhesive patch. In these embodiments, the transdermal drug delivery system may be applied to a skin surface such that the pressure-sensitive adhesive is applied to a skin surface, including an eyelid, by the adhesion of the pressure-sensitive adhesive to the skin surface.

### Support Layer

The transdermal drug delivery system that is employed herein may have a support layer. The support layer may be flexible to an extent that it can be brought into close contact with a skin surface including a front surface of an eyelid. The support is such that it does not absorb the active agent, and does not allow the active agent to be released from the side of the support. The support may include, but is not limited to, non-woven fabrics, woven fabrics, films (including sheets), porous bodies, foamed bodies, paper, composite materials obtained by laminating a film on a non-woven fabric or fabric, and combinations thereof.

Non-woven fabric may include, but is not limited to, the following: polyolefin resins such as polyethylene and polypropylene; polyester resins such as polyethylene terephthalate, polybutylene terephthalate and polyethylene naphthalate; and besides rayon, polyamide, poly(ester ether), polyurethane, polyacrylic resins, polyvinyl alcohol, styrene-isoprene-styrene copolymers, and styrene-ethylene-propylene-styrene copolymers; and combinations thereof. Fabric may include, but is not limited to cotton, rayon, polyacrylic resins, polyester resins, polyvinyl alcohol, and combinations thereof.

The film may include, but is not limited to the following: polyolefin resins such as polyethylene and polypropylene; polyacrylic resins such as polymethyl methacrylate and polyethyl methacrylate; polyester resins such as polyethylene terephthalate, polybutylene terephthalate and polyethylene naphthalate; and besides cellophane, polyvinyl alcohol, ethylene-vinyl alcohol copolymers, polyvinyl chloride, polystyrene, polyurethane, polyacrylonitrile, fluororesins, styrene-isoprene-styrene copolymers, styrene-butadiene rubber, polybutadiene, ethylene-vinyl acetate copolymers, polyamide, and polysulfone; and combinations thereof.

The paper may include, but is not limited to, impregnated paper, coated paper, wood free paper, Kraft paper, Japanese paper, glassine paper, synthetic paper, and combinations thereof. Composite materials may include, but are not limited to, composite materials obtained by laminating the above-described film on the above-described non-woven fabric or fabric.

### Plaster Layer

The transdermal drug delivery system that is employed herein may have a plaster layer provided on the support layer. The plaster layer includes ketotifen freebase, as described in more detail below.

In some embodiments, the plaster layer is an acrylic pressure-sensitive adhesive layer that includes ketotifen freebase. In certain embodiments, the acrylic pressure-sensitive adhesive is a random copolymer of 2-ethylhexyl acrylate and at least one other monomer, e.g., vinyl acetate, butyl acrylate, t-octyl acrylamide and methyl methacrylate.

In some embodiments, the acrylic pressure-sensitive adhesive is a random copolymer of 2-ethylhexyl acrylate and vinyl acetate. In these embodiments, the adhesive may have a composition that is substantially the same as the composition of DuroTak^{®} 87-4098 (National Adhesives, Bridgewater, NJ). The term "substantially the same" as used herein refers to a composition that is a copolymer of 2-ethylhexyl acrylate and vinyl acetate and provides for the storage stable functionality as described above. In some embodiments, the acrylic pressure-sensitive adhesive is DuroTak^{®} 87-4098. DuroTak^{®} 87-4098 is a random copolymer of 2-ethylhexyl acrylate and vinyl acetate pressure-sensitive adhesive.

In some embodiments, the acrylic pressure-sensitive adhesive is a random copolymer of 2-ethylhexyl acrylate, butyl acrylate, t-octyl acrylamide and methyl methacrylate. In these embodiments, the adhesive may have a composition that is substantially the same as the composition of DuroTak^{®} 87-900A (National Adhesives, Bridgewater, NJ). The term "substantially the same" as used herein refers to a composition that is a copolymer of 2-ethylhexyl acrylate, butyl acrylate, t-octyl acrylamide and methyl methacrylate and provides for the storage stable functionality as described above. In some embodiments, the acrylic pressure-sensitive adhesive is DuroTak^{®} 87-900A. DuroTak^{®} 87-900A is a random copolymer of 2-ethylhexyl acrylate, butyl acrylate, t-octyl acrylamide and methyl methacrylate pressure-sensitive adhesive.

In some cases, the plaster layer may further include a plasticizer. In these cases the plasticizer may include, but is not limited to liquid paraffin. The adhesive layer of a subject transdermal delivery system will, in some embodiments, include in addition to the above-discussed components one or more additional components. Additional components of interest include, but are not limited to, a transdermal absorption enhancer, a preservative (e.g., paraben), an antioxidant, a stabilizing agent, a filling agent that contains a hydrophilic polymer; and a cross-linking agent.

In certain embodiments, the plaster layer consists of either DuroTak^{®} 87-4098 or DuroTak^{®} 87-900A as the acrylic pressure-sensitive adhesive and ketotifen freebase as the active agent. In certain embodiments, the plaster layer consists of either DuroTak^{®} 87-4098 or DuroTak^{®} 87-900A as the acrylic pressure-sensitive adhesive, liquid paraffin as a plasticizer, and ketotifen freebase as the active agent. In these embodiments, the amount of either DuroTak^{®} 87-4098 or DuroTak^{®} 87-900A in the plaster layer may range from 60 % to 99 % (w/w), such as from 70 % to 98 % (w/w), and including 80 % to 97 % (w/w). In addition, the amount of liquid paraffin in the plaster layer may range from 0.1 % to 20 % (w/w), such as from 1 % to 8 % (w/w), and including 2 % to 6 % (w/w). In addition, the amount of ketotifen freebase in the plaster layer may range from 0.5 % to 20 % (w/w), such as from 1 % to 10 % (w/w), and including 2 % to 6 % (w/w). In some cases, the plaster layer may have the following composition: DuroTak^{®} 87-4098 in an amount of 91 % (w/w); liquid paraffin in an amount of 5% (w/w); and ketotifen freebase in an amount of 4% (w/w). In some cases, the plaster layer may have the following composition: DuroTak^{®} 87-900A in an amount of 94% (w/w); and ketotifen freebase in an amount of 4% (w/w).

### Active Agent for Ophthalmic Diseases

As reviewed above, the active agent of the subject compositions is ketotifen free base. In certain embodiments, the weight percentage of ketotifen freebase contained in the plaster layer may be in an amount ranging from 0.5 % to 20 % (w/w), such as an amount ranging from 1 % to 10 % (w/w), and including an amount ranging from 2 % to 6 % (w/w). In some cases, the weight percentage of ketotifen freebase contained in the plaster layer is 4% (w/w).

As described above, in certain embodiments, the ketotifen freebase is stable during storage and does not decompose significantly. In some cases, the ratio of the amount of ketotifen freebase in the transdermal drug delivery system to the initial amount of ketotifen freebase in the system is greater than 93% after storage at about 60 °C for at least one month. Also as described above, in certain embodiments, the ketotifen freebase for ophthalmic diseases permeates the skin such that an effective amount of ketotifen freebase is delivered. In some cases, the permeation amount of ketotifen freebase is at least 1 µg/cm² after about 12 hours.

In certain embodiments, the transdermal drug delivery system is prepared as an adhesive patch or an adhesive tape preparation. In these embodiments, the transdermal drug delivery system includes a plaster layer, such as a pressure-sensitive adhesive layer, which contains the active agent for ophthalmic diseases. The plaster layer is provided on a support layer. In some embodiments, a releasable liner is provided on the plaster layer. The releasable liner facilitates the protection of the plaster layer. Prior to application onto a skin surface, the releasable liner may be removed, thereby exposing the plaster layer. The releasable liner may be prepared by treating one side of polyethylene-coated wood free paper, polyolefin-coated glassine paper, a polyethylene terephthalate (polyester) film, a polypropylene film, or the like with a silicone treatment.

In certain cases, the transdermal drug delivery system is an adhesive patch preparation that includes a pressure-sensitive adhesive. In these cases, the transdermal drug delivery system may be prepared in accordance with the solvent coating method,or the like.

In certain embodiments, the transdermal drug delivery system may be prepared using a pressure-sensitive adhesive tape type preparation making use of an acrylic pressure-sensitive adhesive. Typically, the thickness of the plaster layer is about 20 to 300 µm for the pressure-sensitive adhesive type preparation.

### UTILITY

The transdermal drug delivery systems find use in any application where a subject would benefit from being administered ketotifen. In certain embodiments, the systems are employed in the treatment of a condition. By treatment is meant that at least an amelioration of the symptoms associated with the condition afflicting the subject is achieved, where amelioration is used in a broad sense to refer to at least a reduction in the magnitude of a parameter, e.g., symptom, associated with the condition being treated. As such, treatment also includes situations where the pathological condition, or at least symptoms associated therewith, are completely inhibited, e.g., prevented from happening, or stopped, e.g., terminated, such that the subject no longer suffers from the condition, or at least the symptoms that characterize the condition.

In general, administration of ketotifen according to the subject methods can be used to treat diseases or conditions including, but not limited to allergic conjunctivitis, vernal conjunctivitis, chronic conjunctivitis, pollinosis, and the like. Conjunctivitis (commonly called "pink eye") is an inflammation of the conjunctiva (e.g., the outermost layer of the eye and the inner surface of the eyelids), commonly due to an allergic reaction or an infection (usually bacterial, or viral). Pollinosis (also known as "allergic rhinitis" and commonly called "hay fever") is an allergic reaction to pollen or other microscopic substances such as dust mites, mold, animal dander, feathers, and the like.

The transdermal drug delivery system may be used for administering ketotifen to a subject. In these cases, the method includes applying a transdermal drug delivery system, as described herein, to a skin surface of a subject. Subjects may include humans or animals, such as but not limited to mice, rats, dogs, rabbits, and the like.

Also provided is a method for administering an active agent for ophthalmic diseases to an ophthalmic topical tissue of a subject. In some cases, the method includes applying a transdermal drug delivery system, as described herein, to a skin surface including a front surface of an eyelid to administer the ketotifen freebase in the plaster layer to an ophthalmic topical tissue by percutaneous permeation.

The transdermal drug delivery system may be used for treatment of ophthalmic diseases by applying the transdermal drug delivery system to a skin surface, including a front surface of an eyelid to transfer an active agent for ophthalmic diseases in a plaster layer to an ophthalmic topical tissue by percutaneous permeation. The pressure-sensitive adhesive layer of the transdermal drug delivery system can be directly applied to a skin surface, including an eyelid, by the adhesion of the pressure-sensitive adhesive layer to the skin surface.

Also provided is a method for transferring an active agent for ophthalmic diseases to an ophthalmic topical tissue by applying the transdermal drug delivery system to a skin surface including a front surface of an eyelid to transfer the active agent for ophthalmic diseases in a plaster layer by percutaneous permeation to the ophthalmic topical tissue.

In certain embodiments, the transdermal drug delivery system is provided as an adhesive patch and is applied to the skin surface including the front surface of the eyelid, whereby the active agent in the plaster layer can be administered by percutaneous permeation to the ophthalmic topical tissue. When the transdermal drug delivery system is applied to a skin surface, the active agent permeates the skin in contact with the patch to reach an external ophthalmic tissue such as, but not limited to the conjunctiva, lacrimal tissue or cornea.

In some embodiments, the active agent in the plaster layer is administered by percutaneous permeation to the ophthalmic topical tissue without being substantially administered through a systemic blood flow. In these cases, the active agent is administered in such a manner that it is transferred mainly by percutaneous permeation to the external ophthalmic tissue such as, but not limited to the conjunctiva, lacrimal tissue, or cornea from the skin surface, on which the transdermal drug delivery system has been affixed. The efficacy of the transdermal drug delivery system is developed through topical application of the active agent, before a part of the active agent reaches the ophthalmic topical tissue through the systemic blood flow. Accordingly, this does not intend to exclude the fact that a part of the active agent for ophthalmic diseases may be delivered to the ophthalmic topical tissue through the systemic blood flow.

### KITS

Kits for use in practicing certain methods described herein are also provided. In certain embodiments, the kits include a transdermal drug delivery system that includes a support layer and a plaster layer provided on the support layer, wherein the plaster layer contains ketotifen freebase, as described above.

In certain embodiments, the kits will further include instructions for practicing the subject methods or means for obtaining the same (e.g., a website URL directing the user to a webpage which provides the instructions), where these instructions may be printed on a substrate, where substrate may be one or more of: a package insert, the packaging, reagent containers and the like. In the subject kits, the one or more components are present in the same or different containers, as may be convenient or desirable.

The following examples are offered by way of illustration and not by way of limitation.

### EXAMPLES

### I. Analytical procedure used for stability study of ketotifen free base in the formulation

Various Ketotifen patch formulations as described in Table 1 below were prepared. For each formulation, a Ketotifen patch was cut to a 2 cm x 2 cm size. The patch was removed from release liner, then placed into a 50-mL volumetric flask. The polyacrylate adhesive was dissolved by adding 5-mL of tetrahydrofuran (THF) and sonicating for 30 minutes. Then, methanol was added and diluted to volume. The obtained solution was cloudy from the precipitated adhesive. An aliquot of the solution was filtered through a 0.45-µm PTFE filter, then filled into an HPLC vial. The ketotifen concentration in the filtrate was determined by using HPLC. The mobile phase consisted of 0.01 M phosphate buffer (KH₂PO₄) adjusted to pH 8.0 with triethylamine:methanol:acetonitrile (30:15:55, by volume). The mobile phase was delivered at a flow rate of 1.0 ml/min through a C-18 column (4.6 mm I.D.×15 cm, 5 µm) at 40 °C and the detection wavelength was 300 nm.

**Table 1: Stability of Ketotifen**

| Formulation | Condition | Content (±S.D.) |
|---|---|---|
| Example 1 | R.T./1M | 100.9 ± 0.1 |
| Duro Tak 87-900A 96% and ketotifen free base 4% | 60 °C/1M | 94.3 ±1.0 |
| Example 2 | R.T./1M | 101.3 ± 0.7 |
| Duro Tak 87-4098 96% and ketotifen free base 4% | 60 °C/1M | 97.2 ± 2.3 |
| Example 3 | R.T./1M | 100.8 ± 0.9 |
| Duro Tak 87-4098 94% and ketotifen free | 60 °C/1M | 96.3 ± 0.5 |
| base 6% | | |
| Example 4 | R.T./1M | 100.2 ± 0.4 |
| Duro Tak 87-4098 91 % and ketotifen free base 4% and liquid paraffin 5% | 60 °C/1M | 97.0 ± 0.5 |

**Table 2. Comparative Examples**

| Formulation | Condition | Content (±S.D.) |
|---|---|---|
| Comparative Example 1 | R.T./1M | 101.9±0.5 |
| Duro Tak 87-9301 96% and ketotifen free base 4% | 60 °C/1M | 90.4±0.4 |
| Comparative Example 2 | R.T./1M | 100.3±1.1 |
| Duro Tak 87-9088 96% and ketotifen free base 4% | 60 °C/1M | 83.0±0.5 |
| Comparative Example 3 | R.T./1M | 102.1±0.85 |
| Duro Tak 87-2052 96% and ketotifen free base 4% | 60 °C/1M | 100.3±0.9 |
| Comparative Example 4 | R.T./1M | 99.1±0.7 |
| Duro Tak 87-4287 96% and ketotifen free base 4% | 60 °C/1M | 85.9±2.9 |
| Comparative Example 5 | R.T./1M | 90.0±4.9 |
| SIS 25% Hydrogentated Rosin Glycerolester 20% Alicyclic Saturated Hydrocarbon Resin 10% Mineral Oil 41% Ketotefin free base 4% | 60 °C/1M | 73.54±3.0 |

### II. Rat In Vitro Permeation Tests

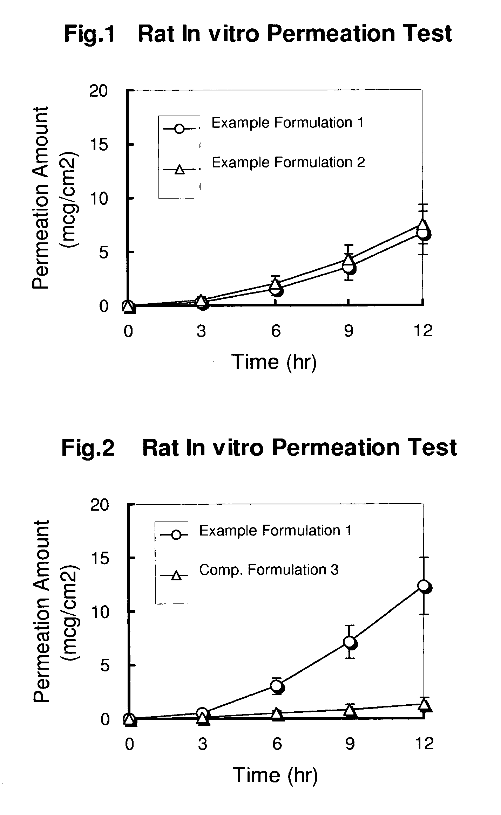

Vertical Franz-type diffusion cells with a diffusional surface area of 1.77 cm² were used to study the permeability of various ketotifen patch formulations. Specifically, Example Formulations 1 and 2 were compared (See FIG. 1), as well as Example Formulation 1 and Comparative Example Formulation 3 (See FIG. 2). Skin samples were obtained from male Wister rats aging 4 to 6 weeks. After hair was shaven using a mechanical hair clipper, without damaging skin, a patch of skin was excised from the dorsal region of each sacrificed rat. The excised rat skins were used as received. They were placed between the donor and receptor compartments of the cells, with the dermal side in direct contact with the receptor medium. Approximately 10 mL of phosphate buffer (pH 7.4) with 20% ethanol was placed in the receptor compartment. Outside the receptor compartment was maintained at 37°C by the water jacket connected to the thermostatic water bath. The medium was stirred at 600 rpm throughout the experiment. The donor compartment contained 11 mg of the sample, i.e., the tape formulations of Example Formulations 1 and 2 reported above and as indicated in FIG. 1; or the tape formulations of Example Formulation 1 and Comparative Example Formulation 3 as reported above and indicated in FIG. 2. Aliquots (0.3 mL) were withdrawn at predetermined time intervals and then the same amount of fresh buffer was added to the receptor compartment to replace what had been removed. The drug concentration was analyzed using HPLC. The results are shown in FIGS. 1 and 2. The results shown in these figures illustrate that Example Formulations 1 and 2 exhibited good permeation profiles while Comparative Example Formulation 3 did not.

## Claims

1. A storage-stable ketotifen freebase transdermal drug delivery system, comprising:
a support layer; and
a plaster layer provided on said support layer comprising ketotifen freebase in an acrylic pressure sensitive adhesive that comprises a copolymer of 2-ethylhexyl acrylate and at least one additional monomer chosen from vinyl acetate, butyl acrylate, t-octyl acrylamide and methyl methacrylate.

2. The transdermal drug delivery system according to Claim 1, wherein said acrylic pressure-sensitive adhesive comprises a random copolymer of 2-ethylhexyl acrylate and vinyl acetate.

3. The transdermal drug delivery system according to Claim 1, wherein said acrylic pressure-sensitive adhesive comprises a random copolymer of 2-ethylhexyl acrylate, butyl acrylate, t-octyl acrylamide and methyl methacrylate.

4. The transdermal drug delivery system according to Claim 1, wherein said plaster layer further comprises a softening agent.

5. The transdermal drug delivery system according to Claim 4, wherein said softening agent is liquid paraffin.

6. The transdermal drug delivery system according to Claim 1 , wherein said plaster layer comprises:
DuroTak^{®} 87-4098 pressure-sensitive adhesive in an amount of 91 % (w/w);
liquid paraffin in an amount of 5% (w/w); and
ketotifen freebase in an amount of 4% (w/w).

7. The transdermal drug delivery system according to Claim 1 , wherein said plaster layer comprises:
DuroTak^{®} 87-900A pressure-sensitive adhesive in an amount of 96% (w/w); and
ketotifen freebase in an amount of 4% (w/w).

8. A kit comprising the transdermal drug delivery system as defined in any one of claims 1 to 7.

## Patentansprüche

1. Lagerstabiles transdermales Wirkstoffabgabesystem für Ketotifen als freie Base, umfassend:
eine Trägerschicht; und
eine Pflasterschicht, die sich auf der Trägerschicht befindet und Ketotifen als freie Base in einem Acryl-Haftkleber, der ein Copolymer von 2-Ethylhexylacrylat und wenigstens einem zusätzlichen Monomer, das aus Vinylacetat, Butylacrylat, t-Octylacrylamid und Methylmethacrylat ausgewählt ist, umfasst, umfasst.

2. Transdermales Wirkstoffabgabesystem gemäß Anspruch 1, wobei der Acryl-Haftkleber ein statistisches Copolymer von 2-Ethylhexylacrylat und Vinylacetat umfasst.

3. Transdermales Wirkstoffabgabesystem gemäß Anspruch 1, wobei der Acryl-Haftkleber ein statistisches Copolymer von 2-Ethylhexylacrylat, Butylacrylat, t-Octylacrylamid und Methylmethacrylat umfasst.

4. Transdermales Wirkstoffabgabesystem gemäß Anspruch 1, wobei die Pflasterschicht weiterhin einen Weichmacher umfasst.

5. Transdermales Wirkstoffabgabesystem gemäß Anspruch 4, wobei es sich bei dem Weichmacher um flüssiges Paraffin handelt.

6. Transdermales Wirkstoffabgabesystem gemäß Anspruch 1, wobei die Pflasterschicht umfasst:
DuroTak^{®}-87-4098-Haftkleber in einer Menge von 91% (w/w);
flüssiges Paraffin in einer Menge von 5% (w/w); und
Ketotifen als freie Base in einer Menge von 4% (w/w).

7. Transdermales Wirkstoffabgabesystem gemäß Anspruch 1, wobei die Pflasterschicht umfasst:
DuroTak^{®}-87-900A-Haftkleber in einer Menge von 96% (w/w); und
Ketotifen als freie Base in einer Menge von 4% (w/w).

8. Kit, umfassend das transdermale Wirkstoffabgabesystem gemäß einem der Ansprüche 1 bis 7.

## Revendications

1. Système d'apport de médicament transdermique de base libre de kétotifène stable au stockage, comprenant :
une couche de support ; et
une couche de sparadrap disposée sur ladite couche de support comprenant de la base libre de kétotifène dans un adhésif autocollant acrylique qui comprend un copolymère d'acrylate de 2-éthylhexyle et au moins un monomère additionnel choisi parmi l'acétate de vinyle, l'acrylate de butyle, le t-octylacrylamide et le méthacrylate de méthyle.

2. Système d'apport de médicament transdermique selon la revendication 1, dans lequel ledit adhésif autocollant acrylique comprend un copolymère statistique d'acrylate de 2-éthylhexyle et d'acétate de vinyle.

3. Système d'apport de médicament transdermique selon la revendication 1, dans lequel ledit adhésif autocollant acrylique comprend un copolymère statistique d'acrylate de 2-éthylhexyle, d'acrylate de butyle, de t-octylacrylamide et de méthacrylate de méthyle.

4. Système d'apport de médicament transdermique selon la revendication 1, dans lequel ladite couche de sparadrap comprend en outre un agent d'adoucissage.

5. Système d'apport de médicament transdermique selon la revendication 4, dans lequel ledit agent d'adoucissage est de la paraffine liquide.

6. Système d'apport de médicament transdermique selon la revendication 1, dans lequel ladite couche de sparadrap comprend :
l'adhésif autocollant DuroTak^{®} 87-4098 dans une quantité de 91 % (p/p) ;
la paraffine liquide dans une quantité de 5% (p/p) ; et
la base libre de kétotifène dans une quantité de 4 % (p/p).

7. Système d'apport de médicament transdermique selon la revendication 1, dans lequel ladite couche de sparadrap comprend :
l'adhésif autocollant DuroTak^{®} 87-900A dans une quantité de 96 % (p/p) ; et
la base libre de kétotifène dans une quantité de 4 % (p/p)·

8. Nécessaire comprenant le système d'apport de médicament transdermique tel que défini dans l'une quelconque des revendications 1 à 7.
